# EUROPEAN PATENT SPECIFICATION

(11) **EP 4 171 376 B1**
(45) Date of publication and mention of the grant of the patent: **06.05.2026**
(21) Application number: 20943271.5
(22) Date of filing: 30.06.2020
(51) Int. Cl.: A61B 5/103

(54) **METHOD FOR DETERMINING A SCALP TYPE OF A USER**
VERFAHREN ZUR BESTIMMUNG DES KOPFHAUTTYPS EINES BENUTZERS
PROCÉDÉ DE DÉTERMINATION D'UN TYPE DE CUIR CHEVELU D'UN UTILISATEUR

(43) Date of publication of application: 03.05.2023
(73) Proprietor: Henkel AG & Co. KGaA, 40589 Düsseldorf (DE)
(72) Inventor: SHI, Yi, Shanghai 200433 (CN); CHANG, Xiaowei, Shanghai 200438 (CN)
(86) International application number: PCT/CN2020/099150
(87) International publication number: WO 2022/000238

(56) References cited:
- WO-A1-2008/035877
- WO-A1-2018/202065
- CN-A- 101 252 880
- CN-A- 101 252 882
- CN-A- 103 971 270
- CN-A- 106 491 086
- CN-A- 109 512 384
- CN-A- 110 074 538
- CN-A- 110 298 393
- CN-U- 201 585 964
- JP-A- 2018 175 909
- JP-B1- 6 647 438
- KR-B1- 100 481 317
- KR-B1- 101 666 955
- KR-B1- 102 090 066

## Description

### TECHNICAL FIELD

The invention relates to the field of skin and hair care, and more particularly to methods for determining the scalp type of a user in order to classify the scalp type into a group associated with suitable skin and hair care product or treatment recommendations. The invention more particularly aims to render the determination of the scalp type more objective to avoid a false assessment of the scalp condition which can lead to the selection of unsuitable scalp or hair care products, and more convenient for a user.

### TECHNOLOGICAL BACKGROUND

Hair care involves the selection of products or treatments compatible with the state of a person's hair. Damaged hair may typically require products that will preserve the structure of hair without inducing further damage. Hair care is not independent from skin care in particular scalp care since the scalp condition may have a strong impact on the general appearance of a person's hairstyle and contribute either to a healthy and appealing look or provide a negative general impression, for example in the event of redness, oiliness, or presence of dandruff.

Despite the relevance of selecting treatments and products that preserve both hair and scalp, there is no general rule for the selection of such products or treatments. Some people typically suffer from a higher sebum secretion on their scalp and therefore would benefit from treatments and products that reduce the level of sebum on their scalp, whereas for other people the opposite occurs.

It is however difficult to make an accurate assessment of one's scalp condition, as this part of the skin generally requires a third party's intervention to be examined and is most of the time hidden under hair.

A visual assessment of a person's scalp condition is generally difficult and results in inaccurate observations. Oiliness and moisture can lead to similar visual assessments although they would not trigger the same treatment recommendation.

In order to assess the scalp type of a user with more accuracy, it is possible to use biophysical measurements of the skin conditions. Typically, electrodes can be used to infer moisture level from the measured conductivity of the skin. Oiliness can be determined as a sebum level using translucent tapes that contact the scalp thereby absorbing sebum. The presence of sebum on the tapes changes their translucency which can be measured optically. Such measurements only provide a set of raw data that can help assess the true condition of the scalp of a user. However, individual measurements are hard to interpret without any comparison means to other scalps. Document JP 6647438 B1 describes a head measurement method that utilizes at least two sensors. The first sensor is a skin moisture content measurement sensor, which acquires data on skin moisture levels. The second sensor is an image sensor, and it captures image data of the area measured by the first sensor.

Document US 20190237194 A1 describes one example of a method for assessing the scalp of a user and recommend a skin treatment product. The assessment of the scalp type relies on measurements of hydration and sebum compiled together with further information provided by the user or by other complementary measurements. All the provided information is converted into a health score to determine a type of scalp treatment.

This approach requires several inputs from a user in order to accurately assess his scalp condition. Such an approach is not convenient for users who do not have much time to spend answering questionnaires or who simply do not know how to answer the questions and add an element of subjectivity to the assessment of the health score. Furthermore, assessing one's scalp type is a continuous process as scalp condition changes after each hair washing step and this may lead to a change in the recommendable hair and/or scalp treatment products or treatments.

A method for simply and objectively assessing the scalp type of user is therefore sought.

### SUMMARY OF THE INVENTION

To address the above need, the invention provides a method for determining a scalp type of a user, the method comprising:
- obtaining a value indicative of a humidity level of a region of the scalp of the user;
- obtaining a value indicative of a sebum level in the region of the scalp of the user;
- comparing a set comprising the obtained value indicative of the humidity level and the value indicative of the sebum level to reference ranges of scalp hydration and sebum levels, the reference ranges forming at least five scalp groups, each scalp group being associated with a humidity level range and a sebum level range, each scalp group being further associated with a corresponding category of skin and/or hair care compositions and/or skin and/or hair care treatments compatible with said humidity level range and sebum level range;
- identifying, based on the comparison, a scalp type of the user among the at least five scalp groups; and
- outputting the identified scalp type.

The invention overcomes the burden of subjectively assessing the scalp type of a user through a visual observation by providing a simple and objectivized approach to determining the scalp type of a user that can be implemented with a portable device and a processor or computer which can be part of the portable device. To enable such an automatic and objectivized assessment of the scalp condition of a user, only two related parameters need to be measured: the humidity level and the sebum level, obtained for a same region of a scalp of a user. Applicant noticed that scalps typically come in five distinct types. These types typically are associated with the type of product or treatment that would best suit the scalp type. Although morphological features within each type might be identical, Applicant noticed that it is possible to exhaustively describe all five scalp types with particular ranges for the humidity and sebum level. The value ranges for humidity and sebum were acquired on a representative batch of users whose scalp type was further measured with optical and biophysical measurement tools. The obtained sebum values and humidity values were plotted in two dimensions and thresholds for each range of sebum and humidity level was found to match five scalp types. Each scalp type is characterized by a particular category of products or treatments recommendable to treat hair or scalp.

The method of the invention consists in comparing measured values for sebum level and humidity level of the scalp of a user to the ranges that were defined on the representative batch of users. This allows a user to know the nature of his scalp condition and chose an appropriate hair or scalp care product and/or hair or scalp treatment.

According to an embodiment, the at least five scalp groups may be established on the basis of data extracted from scalps of at least one hundred users using at least one among:
- measurements of biophysical parameter values of the scalps, the biophysical parameter values comprising a hydration level value and a sebum level value for each scalp;
- an analysis of scalp features on magnified photographs of the scalps of the at least one hundred users,

Optionally, the biophysical parameter values and/or the scalp features may be grouped into five clusters based on similarity of biophysical parameter values and/or scalp features.

According to an embodiment, the value indicative of the humidity level of the region of the scalp of the user and the value indicative of the sebum level of the region of the scalp of the user may be obtained using at least one among:
- measurements of biophysical parameter values of the region of the scalp of the user, the biophysical parameter values comprising a hydration level value and a sebum level value of the region of the scalp of the user;
- an analysis of scalp features on magnified photographs of the region of the scalp of the user.

According to an embodiment, the measurement of biophysical parameter values of the scalps may comprise electrical conductivity measurements or electrical resistance measurements on an upper layer of the scalp to obtain values of hydration levels and measurements of changes of translucency of absorbing strips put in contact with the scalps to obtain sebum levels.

It is to be noted that "electrical conductivity measurements" are equivalent within the meaning of this invention to "electrical resistance measurements". It is to be noted that any other alternative method for measuring the sebum level or humidity level can be used, for example relying on infrared cameras or dedicated measurement devices.

According to an embodiment, the features on the magnified photographs may comprise at least one among:
- an intensity of light reflected by the region of the scalp;
- a count of dandruff on the magnified photograph;
- a count of red portions on the magnified photograph, the red portions being indicative of inflammations on the scalp; and
- an amount of fluorescent light emitted from follicles of the scalp,
wherein each scalp is photographed under natural light, ultraviolet light and polarized light conditions.

Typically such features may be counted per unit area on the images, and the images may either be taken in specific conditions of illumination intensity, viewing angle or distance to the scalp, or be later calibrated to compensate for any differences with respect to reference conditions. Such counts can be implemented on the reference batch of scalps used to set the ranges of humidity and sebum values for all the five scalp groups and also later on on each scalp to be assessed.

According to an embodiment, the method may further comprise:
- defining the at least five scalp groups based on a visual assessment of dryness and oiliness of the scalps of the at least one hundred users, the visual assessment corresponding, for each scalp group of the at least five scalp groups, to an assessment of a similarity in the skin and/or hair care compositions and/or skin and/or hair care treatments adapted for said scalp group.

This optional approach to defining the five scalp groups relies on a professional's assessment of the type of product or treatment that would be recommended for a scalp of a user. The assessment is performed on a representative batch of users and their scalps are grouped according to similarities in the recommendations provided by the professional. Such an approach can also be used in conjunction with the biophysical measurements approach and/or the image analysis approach in order to adjust the threshold of values for each scalp group.

According to an embodiment, the reference ranges may be established on the basis of data extracted from scalps of at least one hundred users, the method further comprising, for each of the at least one hundred users:
- obtaining magnified photographs of the scalps of the at least one hundred users;
- grouping the obtained magnified photographs according to the defined at least five scalp groups;
- for each scalp group of the at least five scalp groups, determining combinations of features found within grouped magnified photographs of scalps belonging to said scalp group.

Such an approach consists in a learning process during which features on images are identified based on scalps that have already been classified into each of the least five scalp groups. The process can for example be implemented by a human eye counting features on the magnified photographs to determine the combination of features of the scalps that can be used to match each scalp group that was already defined with ranges of humidity levels and sebum levels.

The at least five scalp groups comprise:
- a first scalp group corresponding to a hydration level measurable as an upper skin conductivity of 0 µS to 40 µS and a Sebum level measurable as a reduction in translucency of a sebum-absorbing tape film that had been in contact with the scalp of the user between 0% and 20%;
- a second scalp group corresponding to a hydration level measurable as an upper skin conductivity of 0 µS to 80 µS and a Sebum level measurable as a reduction in translucency of a sebum-absorbing tape film that had been in contact with the scalp of the user between 20% and 60%;
- a third scalp group corresponding to a hydration level measurable as an upper skin conductivity of 0 µS to 80 µS and a Sebum level measurable as a reduction in translucency of a sebum-absorbing tape film that had been in contact with the scalp of the user between 60% and 100%;
- a fourth scalp group corresponding to a hydration level measurable as an upper skin conductivity of 40 µS to 80 µS and a Sebum level measurable as a reduction in translucency of a sebum-absorbing tape film that had been in contact with the scalp of the user between 0% and 40%;
- a fifth scalp group corresponding to a hydration level measurable as an upper skin conductivity at or above 80 µS and a Sebum level measurable as a reduction in translucency of a sebum-absorbing tape film that had been in contact with the scalp of the user between 0% and 60%.

According to embodiment, the scalp groups may further comprise the following features:
- the first scalp group is associated with a corresponding category of skin and/or hair care compositions comprising between 10% and 18% surfactants, and have a Zein value lower than 0,75;
- the second scalp group is associated with a corresponding category of skin and/or hair care compositions comprising between 12% and 20% surfactants, and have a Zein value lower than 1;
- the third scalp group is associated with a corresponding category of skin and/or hair care compositions comprising between 14% and 20% surfactants, and have a Zein value lower than 2,5;
- the fourth scalp group is associated with a corresponding category of skin and/or hair care compositions comprising between 10% and 18% surfactants, and have a Zein value lower than 2; and
- the fifth scalp group is associated with a corresponding category of skin and/or hair care compositions comprising between 10% and 18% surfactants, and have a Zein value lower than 1,5.

According to an embodiment, the scalp groups may further comprise the following features:
- the first scalp group is associated with a corresponding category of skin and/or hair care compositions comprising between 0% and 2% of an anti-dandruff agent;
- the second scalp group is associated with a corresponding category of skin and/or hair care compositions comprising between 0% and 2% of an anti-dandruff agent;
- the third scalp group is associated with a corresponding category of skin and/or hair care compositions comprising between 0% and 1% of an anti-dandruff agent;
- the fourth scalp group is associated with a corresponding category of skin and/or hair care compositions void of anti-dandruff agent; and
- the fifth scalp group is associated with a corresponding category of skin and/or hair care compositions void of anti-dandruff agent.

The method of the invention further pertains to a method for recommending a skin or hair care product and/or a skin or hair care treatment to a user comprising:
- determining a scalp type of the user with a method as described above;
- determining, based on the identified scalp type of the user, the corresponding category of skin and/or hair care compositions and/or skin and/or hair care treatments compatible with the humidity level range and sebum level range of the scalp type of the user; and
- outputting the corresponding category of skin and/or hair care compositions and/or skin and/or hair care treatments.

This method may advantageously be implemented with a processor, part of a portable device or a computer. Information regarding the category of skin and/or hair care compositions and/or skin and/or hair care treatments compatible with the humidity level range and sebum level range of the scalp type of the user may be stored on a local database or accessed at a distant computer/server or in a cloud environment. The database may also be updated using either user feedback or to take into account evolutions and improvements in the matching method described above.

According to an embodiment, the method for recommending a skin or hair care product and/or a skin or hair care treatment to a user may further comprise:
- obtaining a magnified photograph of the region of the scalp of the user;
- upon identifying a number of red portions on the magnified photograph that is above a predetermined threshold, recommend a skin and/or hair care composition comprising a reduced proportion of surfactants adapted for sensitive scalps.

According to an embodiment, the method for recommending a skin or hair care product and/or a skin or hair care treatment to a user may further comprise:
- comparing a current value indicative of the humidity level of the region of the scalp of the user and a current value indicative of the sebum level of a region of the scalp of the user to previously obtained values for the region of the scalp of the user;
- upon determining that the sebum level of the region of the scalp of the user increases, output a recommendation to increase the frequency of hair wash using a hair care product compatible with the scalp type of the user;
- upon determining that the sebum level of the region of the scalp of the user decreases, output a recommendation to use a hair or scalp care product comprising a lower proportion of surfactants.

In such a dynamic process of determining the evolution over time of the scalp of the user suitable products or treatment recommendations may differ from those normally recommended for a given scalp type, in order to stimulate a desired change of the scalp condition into a condition preset by the user or recommended by a hair care professional for example.

The invention also pertains to a computer program product comprising instructions for executing a method for determining a scalp type of a user, the method comprising:
- obtaining, as an input, a value indicative of a humidity level of a region of the scalp of the user;
- obtaining, as an input, a value indicative of a sebum level in the region of the scalp of the user;
- comparing a set comprising the obtained value indicative of the humidity level and the value indicative of the sebum level to reference ranges of scalp hydration and sebum levels, the reference ranges forming at least five scalp groups, each scalp group being associated with a humidity level range and a sebum level range, each scalp group being further associated with a corresponding category of skin and/or hair care compositions and/or skin and/or hair care treatments compatible with said humidity level range and sebum level range;
- identifying, based on the comparison, a scalp type of the user among the at least five scalp groups; and
- outputting the identified scalp type.

In other words, the invention concerns a non-transitory computer readable storage medium having stored thereon a computer program comprising instructions for execution of the method described above.

### BRIEF DESRIPTION OF THE DRAWINGS

The present disclosure will hereinafter be described in conjunction with the following drawing figures, wherein like numerals denote like elements, and:
Fig. 1 shows a simplified workflow of a possible method according to an exemplary embodiment;
Fig. 2 is a schematic representation of methods for measuring physiological parameters of the scalp of a user according to an embodiment;
Fig. 4 is a diagram showing a distribution of points corresponding to humidity levels (horizontal axis) and sebum levels (vertical axis) measured on a batch of users, showing a clustering of the points into five scalp groups;
Fig. 4a is a schematic representation of method for acquiring images of the scalp of a user for features analysis thereof;
Fig. 4b is a schematic representation of a picture of the scalp of a user showing feature that can be found thereon.

### DETAILED DESCRIPTION

The invention provides a method for determining the scalp type of a user using a minimal amount of parameters from the scalp of the user and based on an objective assessment of the scalp condition.

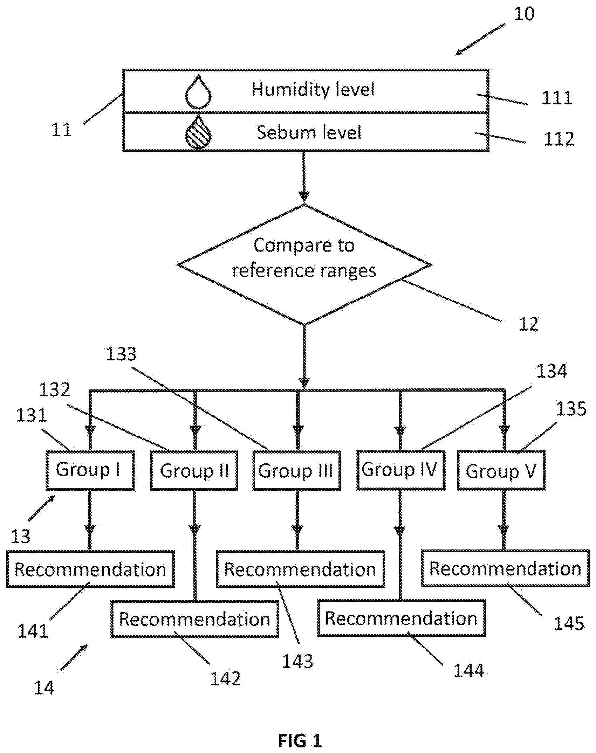

As seen on figure 1, the method 10 for determining a scalp type of a user starts with a step 11 by obtaining a hydration level 111 and a sebum level 112 on a region of a scalp of a user. There is no limitation to the size or location of the region of the scalp of a user, however it is preferable to choose a section of the scalp where hair density does not prevent measuring devices from accessing the skin on the head of the user. Regardless of the unit in which the sebum level and the hydration level are expressed , these may advantageously be converted into a conductivity value equivalent for the hydration level value, for example per unit area of the scalp surface, and a percentage of light transmissivity for the sebum level value. The light transmissivity value describes the percentage of reduction in light that can pass through a standard strip of paper put into contact with the scalp of the user to soak sebum present thereon. Other units can be used to describe the ranges and features of each scalp group. Values for these two parameters that describe the greasiness of the scalp (via the sebum level value) and the moisture of the scalp (via the humidity level value) are compared at step 12 to reference ranges for both parameters.

These reference ranges define at least five scalp groups 131-135, which are defined based on the type of treatments or products (in particular categories of skin and/or hair care compositions and/or skin and/or hair care treatments 141-145) that would typically be suitable or recommendable to be used with each scalp group 131-135. Applicant noticed that sebum and humidity are two particularly suitable parameters to assess a scalp type. This observation comes from a thorough study of more than one hundred representative users of different ages and genders.

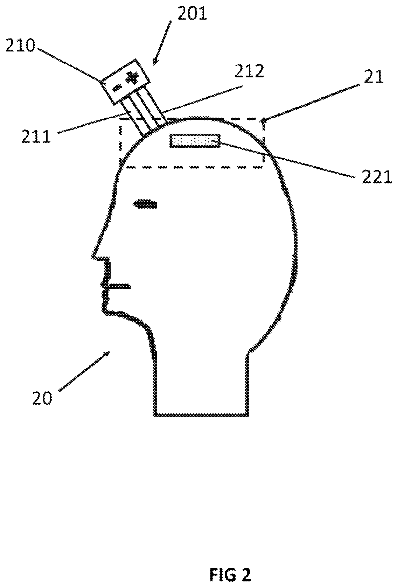

On this representative batch of users, hydration levels of the scalp were measured using a measuring device typically comprising electrodes as represented on figure 2. Such a device 201 comprises at least two electrodes 211, 212 and a controller 210 for measuring a current that passes through the electrodes and region of the scalp 21 of the user 20. Such a device gives access to the hydration levels of the stratum corneum layer of the scalp and outputs a value in Siemens (unit S or µS) representative of the scalp conductivity per unit area of scalp.

As further seen on figure 2, a measurement of sebum levels can be obtained by applying a standard strip 221 of paper on the region of the scalp 21 of the user 20. By absorbing sebum present on the scalp, the strip 221 of paper will see a change in its translucency. 0% indicates no change translucency (indicative of the lowest possible sebum concentration on the scalp of a user, so that the translucency of the standard strip of paper does not change at all with respect to a strip that was not in contact with the scalp) and 100 % indicates a full decrease in translucency compared to a strip with no sebum thereon (indicative of a highest measurable sebum level on the scalp of the user).

Other standards for defining and measuring the scalp humidity level and scalp sebum level can be used.

Further instruments can also be used to measure trans-epidermal water loss on the scalp, pH values of the scalp, in order to obtain a fuller picture of the condition of the scalp of the users 20 from the representative batch of users.

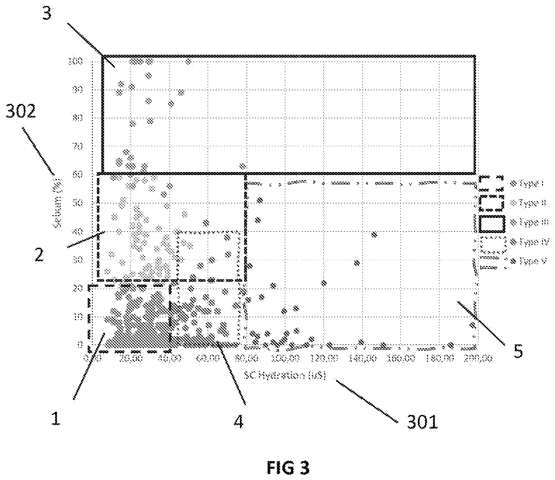

Once these parameters are measured for all users 20 of the representative batch of users, the values of sebum level versus humidity level are plotted in a two-dimensional diagram as represented on figure 3.

Vertical axis 302 on figure 3 shows sebum level values in arbitrary units (corresponding to the translucency measured with a standard strip of paper as explained above). These units can be converted into any other unit depending on the type of method used for determining the sebum level.

Horizontal axis on figure 3 shows the hydration level expressed as a conductivity of the stratum corneum measured in microSiemens (µS). These units can be converted into any other unit depending on the type of method used for determining the hydration level.

As can be seen on figure 3, the distribution of data points is not homogenous across all the two-dimensional area of the graph. Most users have moderately low sebum and hydration levels of their scalps, which would generally be considered as a "normal" scalp condition, or a scalp condition that would naturally occur shortly after washing one's hair.

Each individual scalp condition represented on figure 3 is also separately assessed with a hair care professional to determine the most suitable range of categories of products for hair or scalp treatment. From this assessment and from using a clustering algorithm, it has been found that scalp types can be divided into the at least five groups shown on figure 3.

A first scalp group 1 typically corresponds to hydration levels measurable as an upper skin conductivity of 0 µS to 40 µS and sebum levels measurable as a reduction in translucency of a sebum-absorbing tape film that had been in contact with the scalp of the user between 0% and 20%. This group in figure 3 encompasses about 47% of all user scalp types. This group typically corresponds to scalps that are not very moist and not very greasy.

A second scalp group 2 typically corresponds to hydration levels measurable as an upper skin conductivity of 0 µS to 80 µS and sebum levels measurable as a reduction in translucency of a sebum-absorbing tape film that had been in contact with the scalp of the user between 20% and 60%. One notices on figure 3 that some points from the second scalp group 2 seem to partially overlap with points that would typically be graded by hair care professionals as being suitable for other categories of scalp or hair care products and/or scalp and/or hair care treatments, namely those of scalp group 4. This is not a matter for concern as the boundaries between adjacent scalp group types would not necessarily lead to incompatible recommendations. The scalps of the second scalp group 2 typically correspond to scalps that are not very moist and slightly greasy. A total of about 15% of all scalps observed in the representative batch fall into this scalp group. One frequent evolution that can for example be observed when a user does not wash his hair for some time is a tendency to move from the first scalp group to the second scalp group. Therefore, based on the frequency on which a person washes his hair, different types of hair or scalp products might be suitable for ensuring the scalp stays healthy. It is further to be noted that a healthy scalp typically has moisture and greasiness values that fit the values observed for the first scalp group 1.

A third scalp group 3 typically corresponds to hydration levels measurable as an upper skin conductivity of 0 µS to 80 µS and sebum levels measurable as a reduction in translucency of a sebum-absorbing tape film that had been in contact with the scalp of the user between 60% and 100%. As seen on figure 3, the actual boundary in terms of measured hydration levels can be extended to conductivities above 80 µS, although frequency of occurrence of such extreme cases is very low. Scalps of the third scalp group 3 typically corresponds the greasiest scalps observed and encompass about 8% of all scalps observed in the experiments conducted by the Applicant. This would be considered as not esthetical to the naked eye and would therefore be associated with hair or scalp care products that significantly reduce oiliness of skin. Such products would generally be considered as too aggressive and potentially slightly damageable to the hair in other scalp types but would not affect the scalp or hair of users from the third scalp group 3.

A fourth scalp group 4 typically corresponds to hydration levels measurable as an upper skin conductivity of 40 µS to 80 µS and sebum levels measurable as a reduction in translucency of a sebum-absorbing tape film that had been in contact with the scalp of the user between 0% and 40%. Some of the points on the diagram of figure 3 belong simultaneously to the type of the second scalp group 2 and the type of the fourth scalp group, especially for sebum levels between 20% and 40% and hydration levels expressed as a conductance between 40 µS and 80 µS. As explained above, this does not have a significant impact on the product recommendation as at these intermediate values the wish to clean hair in order to reduce greasiness is the general recommendation in terms of scalp and/or hair care treatments or products. No further action on hydration is deemed to be required as hydration is not esthetically perceived as being a sign of unhealthy or dirty hair or scalp. About 22% of all users had a scalp type belonging to the fourth scalp group 4.

Finally, figure 3 shows a fifth scalp group 5 corresponding typically to hydration levels measurable as an upper skin conductivity at or above 80 µS and sebum levels measurable as a reduction in translucency of a sebum-absorbing tape film that had been in contact with the scalp of the user between 0% and 60%. This group encompasses about 8% of all scalp groups observed in the experiment conducted by the Applicant.

It could be summarized that users whose scalp is identified as belonging to the third scalp group 3 would wish to reduce the greasiness of their hair. **It** could be that this greasiness is only a temporary result of a longer than usual period spent without washing one's hair or a temporary change in diet. Users whose scalp type is determined as belonging to the fifth scalp group 5 may wish to reduce the moist level of their scalp. A sweaty scalp is typically considered as more likely to attract residues from grooming or polluting particles. **It** is generally admitted that products with more surfactant content are best suited for such scalp types (with 10% to 18% surfactants for example).

Another aspect of the invention concerns a time-dependent monitoring of the evolution of the scalp type of one user. A user whose scalp type is identified as on average belonging to one of the first scalp group 1, second scalp group 2 or fourth scalp group 4 most of the time, would be surprised to notice a sudden change in his scalp group if it switches to the third or fifth scalp groups. This could trigger further awareness that an unusual change in either diet, daily routine or a particular health condition is affecting the scalp of the user. One recommendation, beyond hair or scalp treatments or application of specific hair or scalp care products could also be seeking advice from a professional or provision of specific advice on diet or routine fitness activities that could affect moisture or greasiness of scalps.

A user's scalp type can therefore be easily determined by comparing a set of values comprising a hydration level value and a sebum level value of the region of the scalp 21 of the user 20 to references ranges as those shown on the diagram of figure 3.

**It** is to be noted that, besides the measurement of these two biophysical parameters to determine the scalp type of a user, it is also possible to analyze the content of images of the scalp of a user.

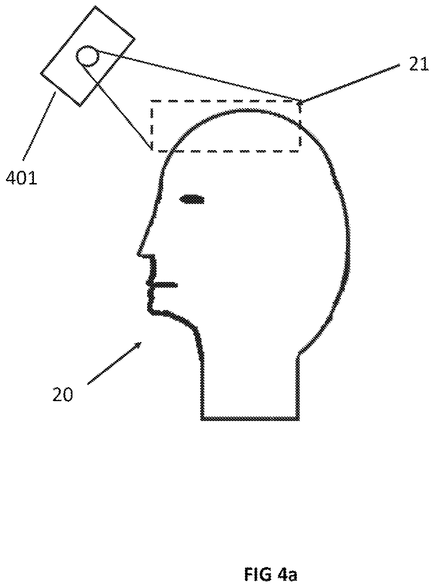

Figure 4a is a schematic drawing of an image acquisition device 401 capturing light reflected from the region of the scalp 21 of a user 20. Although not represented, the acquisition device 401 may typically comprise a light emitting unit that is capable of casting ultraviolet light (with wavelengths ranging from 100 nm to 400 nm) or polarized light (linearly polarized light in particular) onto the region of the scalp 21 of the user 20. A source of visible light (with wavelengths typically ranging from 400 nm to 800 nm) may also be present. A corresponding sensor unit is also included in the image acquisition device 401. Image acquisition device 401 also advantageous takes magnified images of the region of the scalp 21 of the user 20, for example with a magnification factor of about 80.

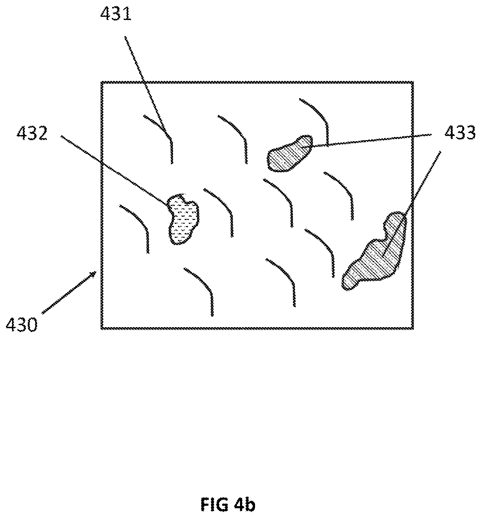

An example of a magnified image 430 of the region of the scalp 21 of the user 20 is shown on figure 4b. This magnified image 430 comprises hairs 431, red portions 432 and signs of dandruff 433.

Typically, image analysis consists in looking at the intensity of visible light reflected from the scalp on the image to determine the humidity level of the scalp. A flaky state of hair, with the presence of dandruff 433 typically is a sign of low moisture of the scalp. Based on the number of dandruff 433 portions (their size and number over the magnified image 430) it is possible to qualitatively grade the humidity level as high, slight or low.

Polarized images emphasize micro-inflammation or higher sensitivity areas of the scalp. Erythema, appearance of blood capillaries typically seen as red portions 432 are signs of a higher sensitivity. Based on the amount of these areas (their respective size over the whole magnified image 430) a qualitative score such as high, slight, or low can be given to the scalp.

Under ultra-violet (UV) light, follicular clogging can be detected. Orange or red fluorescence areas in the vicinity of hair roots can be seen on the magnified images (not shown). This fluorescence is typically due to porphyrin fluorescence under UV light illumination. Porphyrins are produced by lipid-fed bacteria called Cutibacterium Acnes, which therefore gives insights into the sebum level of the scalp. Follicular clogging (and sebum level) can qualitatively be graded as high, slight or low from the magnified image 430.

Table 1 shown below summarizes the features that can typically be found in each scalp groups defined above using either the biophysical parameters defined above or the image analysis description.

**Table 1: correspondence between reference ranges from biophysical measurement of humidity level and sebum level and features count on images across all five scalp groups**

| **Scalp groups** | Biophysical parameter-based Classification | Imaging based Classification |
|---|---|---|
| Group 1 | Low hydration level (0-40 µS) | slight to high dandruff level |
| Low moisture | Low sebum level (0 - 20%) | |
| Low greasiness | | Low reflectivity |
| Group 2 | Low hydration level (0-80 µS), | Slight fluorescence no-to-slight dandruff level |
| Low moisture | Moderately high sebum level (20% - 60%) | |
| Moderately high greasiness | | |
| Group 3 | Low hydration level (0-80 µS, > 80 µS), | high fluorescence, no-slight dandruff |
| Low moisture | Extremely high sebum level (> 60%) | |
| Extremely high greasiness | | |
| Group 4 | Moderately high hydration level (40 µS -80 µS), | Moderately high reflectivity, |
| Moderately high moisture | | |
| | Low sebum level (0-40%) | Low-medium fluorescence |
| Low greasiness | | |
| | | No-slight dandruff |
| Group 5 | Extreme high hydration level (> 80 µS), | High reflectivity, no dandruff |
| Extremely high moisture | Low sebum level (0 -60%) | |
| Low greasiness | | |

Now that a method for determining the scalp type of a user has been described, a further method using the determined scalp type to recommend a skin or hair care product and/or a skin or hair care treatment to a user is described.

As explained above. Each scalp group is defined based on the category of skin and/or hair care compositions and/or skin and/or hair care treatments compatible with the humidity level range and sebum level observed in that group.

Typically, for users that have a scalp type belonging to the first scalp group 1, it is sought to increase scalp hydration to avoid abnormal desquamation and in order to reduce scalp sensitivity.

To achieve this, surfactants used in shampoo formulas for this scalp type should be mild, avoid irritation to the scalp barrier. The total level of surfactants should not exceed a certain threshold to avoid further excessive reduction of sebum from the scalp. Anti-dandruff agents may be used to cure flakes and maintain a healthy scalp environment. Typical ranges would therefore comprise 10% to 18% surfactants, a total amount of anti-dandruff agent between 0% and 2% and a Zein value inferior to 0,75. The Zein value defines the number of grams of zein that can be dissolved by every gram of surfactant. Zein is a corn protein that is considered to be similar to keratin present in the skin and hair. Zein value is an indicator of the irritation potential of a surfactant or surfactant-based product.

Typically, for users that have a scalp type belonging to the second scalp group 2, it is sought to improve the water- oil imbalance on the scalp, by increasing the moisture level and or reducing the sebum level, so that the redness, sensitivity of the scalp decreases.

To achieve this, surfactants used in shampoo formulas for this scalp type should offer a balance between mildness for the scalp and sufficient cleaning efficiency of the shampoo. Anti-dandruff agents may be used to cure flakes and maintain a healthy scalp environment. Typical ranges would therefore comprise 12% to 20% surfactants, a total amount of anti-dandruff agent between 0% and 2% and a Zein value inferior to 1.

Typically, for users that have a scalp type belonging to the third scalp group 3, it is sought to reduce the sebum concentration on the scalp of the user. Sufficient cleaning with more surfactants is therefore recommended to avoid severe follicular clogging, unhealthy follicles irritations and greasy, sticky hair.

To achieve this, the total level of surfactants be high enough. Some anti-dandruff agents may also be present as long as they do not reduce the cleaning efficiency conferred by the surfactants. Typical ranges would therefore comprise 14% to 20% surfactants, a total amount of anti-dandruff agent between 0% and 1% and a Zein value inferior to 2,5.

It is generally recommended to reassess the scalp type of a user within 24 hours after washing one's hair to avoid any significant influence of the accumulated dirtiness of the hair and scalp on the assessment of the scalp type of the user.

Typically, for users that have a scalp type belonging to the fourth scalp group 4, no special effect is sought other than maintaining values within the ranges of this scalp group. Scalps of this scalp group are generally strongly resilient to external influences (chemical treatments of hair, hair coloration, weather conditions for example).

As a result, there is no special restriction as to the type of surfactants or their total level to be used, and anti-dandruff agents re generally not required. Typical ranges would therefore comprise 10% to 18% surfactants and a Zein value inferior to 2.

Typically, for users that have a scalp type belonging to the fifth scalp group 5, it is sought to achieve a better cleaning of the scalp to remove any pollution particuls or residues from grooming that accumulate on sweaty scalps, and avoid proliferation of micro-organisms on the scalp that can lead to irritations that render the scalp more sensitive.

To achieve this, some surfactants should be present to ensure proper cleansing of the scalp but they should be moderately mild. Anti-dandruff agents are optional. Typical ranges would therefore comprise 10% to 18% surfactants and a Zein value inferior to 1,5.

The above categories of product or treatment recommendations can be further enhanced by adding information from an analysis of features on a magnified image of the scalp of the user.

Indeed, a thorough count of these features can allow a finer tuning of each of the above recommendations to make an even more personalized scalp and hair care assessment of the scalp of the user.

For example, in the event of high sensitivity shown as a high number of red areas on the scalp, it is recommended to put a stronger focus on the mildness of the surfactants used in the shampoo.

Anti dandruff agents can be increased or decreased in concentration in the final shampoo formula based on the amount of dandruff seen on the magnified images. As for follicular clogging (seen as a higher or lower presence of fluorescent traces on images under UV light illumination, which reflects a higher or lower sebum concentration), they are not only representative of sebum concentration but are an indicator of the interaction between hair and scalp at the hair roots. In case of a high number of follicular clogging, it would be recommended to increase the frequency of hair washing, and to adjust the surfactant concentration (by increasing its concentration for higher follicular clogging levels), to reduce the number of follicular clogging to better preserve hair.

Surfactant used in shampoo can typically be selected among anionic surfactants, non-anionic surfactants, amphoteric surfactants.

Anionic surfactants can typically comprise at least one among: Acylated Amino Acid such as for example Sodium Lauroyl Sarcosinate or TEA-Cocoyl Glutamate; Carboxylic Acids and salts such as for example Sodium Laureth-13 Carboxylate; sulfonic Acid derivatives such as for example Sodium Methyl Cocoyl Taurate, Sodium Cocoyl Isethionate or Sodium C14-16 Olefin Sulfonate ; Sulfuric Acid derivatives such as for example Sodium Laureth Sulfate or Ammonium Lauryl Sulfate.

Non-anionic surfactants can typically comprise at least one among: Alkyl Glucosides: such as for example Coco-Glucoside or Lauryl Glucoside; Amine oxides such as for example Lauramine Oxide.

Amphoteric surfactants can typically comprise at least one among: Alkyl amido Alkyl Amines such as for example Sodium Lauroamphoacetate; Alkyl Betaines such as for example Cocamidopropyl betaine or Coco-betaine, coco-hydroxysultaine.

Typical anti-dandruff (AD) agents that could be found in shampoo include one among: Zinc pyrithione, Piroctone Olamine, Ketoconazole, Climbazole, Hexamidine diisethionate or Apium Graveolens (Celery) Seed Extract for example.

The following table 2 summarizes the different recommendations described above.

**Table 2: type of typically shampoo compositions suitable for each scalp group further based on features extracted from an image analysis of the scalp.**

| **5 different Scalp Conditions** | Sensitivity: "high" | Sensitivity: "high" | Sensitivity: "Slight" / "no" | Sensitivity: "Slight" / "no" |
|---|---|---|---|---|
| | Dandruff: "high"/ "slight" | Dandruff: "No" | Dandruff: "high"/ "slight" | Dandruff: "No" |
| Group 1 Low moisture, Low greasiness | - Surfactants: 10%~18% | - Surfactants: 10%~18% | - Surfactants: 10%~18% | Surfactants: 10%~18% |
| | - Zein: <0.5 | Zein: <0.5 | - Zein: <0.75 | Zein: <0.75 |
| | - AD: 0.2%~2% | - AD: 0%~0.5% | - AD: 0.2%~2% | - AD: 0%~0.5% |
| Group 2 Low moisture, Moderately high greasiness | - Surfactants: 12%~20% | - Surfactants: 12%~20% | - Surfactants: 12%~20% | Surfactants: 12%~20% |
| | - Zein: <0.5 | - Zein: <0.5 | - Zein: <1 | - Zein: <1 |
| | - AD: 0.2%~2% | - AD: 0%~0.5% | - AD: 0.2%~2% | - AD: 0%~0.5% |
| Group 3 Low moisture, Extremely high greasiness | - Surfactants: 14%~20% | - Surfactants: 14%~20% | - Surfactants: 14%~20% | Surfactants: 14%~20% |
| | - Zein: <1 | - Zein: <1 | - Zein: <2.5 | - Zein: <2.5 |
| | - AD: 0.2%~2% | - AD: 0%~0.5% | - AD: 0.2%~2% | - AD: 0%~0.5% |
| Group 4 Moderately high moisture, Low greasiness | - Surfactants: 10%~18% | - Surfactants: 10%~18% | - Surfactants: 10%~18% | Surfactants: 10%~18% |
| | - Zein: <1 | - Zein: <1 | - Zein: <2 | |
| | - AD: | - AD: / | - AD: 0.2%~2% | - Zein: <2 |
| | 0.2%~2% | | | - AD: / |
| Group 5 Extremely high moisture, Low greasiness | - Surfactants: 10%~18% | - Surfactants: 10%~18% | - Surfactants: 10%~18% | Surfactants: 10%~18% |
| | - Zein: <1 | - Zein: <1 | - Zein: <1.5 | |
| | - AD: 0.2%~2% | - AD: / | - AD: 0.2%~2% | - Zein: <1.5 |
| | | | | - AD: / |

## Claims

1. A method (10) for determining a scalp type of a user (20), the method comprising:
- obtaining a value indicative of a humidity level (111) of a region of the scalp (21) of the user;
- obtaining a value indicative of a sebum level (112) in the region of the scalp of the user;
- comparing a set comprising the obtained value indicative of the humidity level and the value indicative of the sebum level to reference ranges of scalp hydration levels and sebum levels, the reference ranges forming at least five scalp groups, each scalp group being associated with a humidity level range and a sebum level range, each scalp group being further associated with a corresponding category of skin and/or hair care compositions and/or skin and/or hair care treatments compatible with said humidity level range and sebum level range;
wherein the at least five scalp groups comprise:
- a first scalp group (1) corresponding to a hydration level measurable as an upper skin conductivity of 0 µS to 40 µS and a Sebum level measurable as a reduction in translucency of a sebum-absorbing tape film that had been in contact with the scalp of the user between 0% and 20%;
- a second scalp group (2) corresponding to a hydration level measurable as an upper skin conductivity of 0 µS to 80 µS and a Sebum level measurable as a reduction in translucency of a sebum-absorbing tape film that had been in contact with the scalp of the user between 20% and 60%;
- a third scalp group (3) corresponding to a hydration level measurable as an upper skin conductivity of 0 µS to 80 µS and a Sebum level measurable as a reduction in translucency of a sebum-absorbing tape film that had been in contact with the scalp of the user between 60% and 100%;
- a fourth scalp group (4) corresponding to a hydration level measurable as an upper skin conductivity of 40 µS to 80 µS and a Sebum level measurable as a reduction in translucency of a sebum-absorbing tape film that had been in contact with the scalp of the user between 0% and 40%;
- a fifth scalp group (5) corresponding to a hydration level measurable as an upper skin conductivity at or above 80 µS and a Sebum level measurable as a reduction in translucency of a sebum-absorbing tape film that had been in contact with the scalp of the user between 0% and 60%;
- identifying, based on the comparison, a scalp type of the user among the at least five scalp groups (131-135); and
- outputting the identified scalp type.

2. The method according to claim 1, wherein the at least five scalp groups are established on the basis of data extracted from scalps of at least one hundred users using at least one among:
- measurements of biophysical parameter values of the scalps, the biophysical parameter values comprising a hydration level value and a sebum level value for each scalp;
- an analysis of scalp features on magnified photographs (430) of the scalps of the at least one hundred users,

3. The method according to any one of the preceding claims, wherein the value indicative of the humidity level of the region of the scalp of the user and the value indicative of the sebum level of the region of the scalp of the user are obtained using at least one among:
- measurements of biophysical parameter values of the region of the scalp of the user, the biophysical parameter values comprising a hydration level value and a sebum level value of the region of the scalp of the user;
- an analysis of scalp features on magnified photographs of the region of the scalp of the user.

4. The method according to any one of claims 2 to 3, wherein the measurement of biophysical parameter values of the scalps comprises electrical conductivity measurements or electrical resistance measurements on an upper layer of the scalp to obtain values of hydration levels and measurements of changes of translucency of absorbing strips put in contact with the scalps to obtain sebum levels.

5. The method according to any one of claims 2 to 4 wherein the features on the magnified photographs comprise at least one among:
- an intensity of light reflected by the region of the scalp;
- a count of dandruff (433) on the magnified photograph;
- a count of red portions (432) on the magnified photograph, the red portions being indicative of inflammations on the scalp; and
- an amount of fluorescent light emitted from follicles of the scalp,
wherein each scalp is photographed under natural light, ultraviolet light and polarized light conditions.

6. The method according to any one of claims 2 to 5, further comprising:
- defining the at least five scalp groups (1-5) based on a visual assessment of dryness and oiliness of the scalps of the at least one hundred users, the visual assessment corresponding, for each scalp group of the at least five scalp groups, to an assessment of a similarity in the skin and/or hair care compositions and/or skin and/or hair care treatments adapted for said scalp group.

7. The method according to any one of the preceding claims, wherein the reference ranges are established on the basis of data extracted from scalps of at least one hundred users, the method further comprising, for each of the at least one hundred users:
- obtaining magnified photographs of the scalps of the at least one hundred users;
- grouping the obtained magnified photographs according to the defined at least five scalp groups;
- for each scalp group of the at least five scalp groups, determining combinations of features found within grouped magnified photographs of scalps belonging to said scalp group.

8. The method according to any one of the preceding claims, wherein
- the first scalp group is associated with a corresponding category of skin and/or hair care compositions comprising between 10% and 18% surfactants, and have a Zein value lower than 0,75;
- the second scalp group is associated with a corresponding category of skin and/or hair care compositions comprising between 12% and 20% surfactants, and have a Zein value lower than 1;
- the third scalp group is associated with a corresponding category of skin and/or hair care compositions comprising between 14% and 20% surfactants, and have a Zein value lower than 2,5;
- the fourth scalp group is associated with a corresponding category of skin and/or hair care compositions comprising between 10% and 18% surfactants, and have a Zein value lower than 2; and
- the fifth scalp group is associated with a corresponding category of skin and/or hair care compositions comprising between 10% and 18% surfactants, and have a Zein value lower than 1,5.

9. The method according to any one of the preceding claims, wherein:
- the first scalp group is associated with a corresponding category of skin and/or hair care compositions comprising between 0% and 2% of an anti-dandruff agent;
- the second scalp group is associated with a corresponding category of skin and/or hair care compositions comprising between 0% and 2% of an anti-dandruff agent;
- the third scalp group is associated with a corresponding category of skin and/or hair care compositions comprising between 0% and 1% of an anti-dandruff agent;
- the fourth scalp group is associated with a corresponding category of skin and/or hair care compositions void of anti-dandruff agent; and
- the fifth scalp group is associated with a corresponding category of skin and/or hair care compositions void of anti-dandruff agent.

10. A method for recommending a skin or hair care product and/or a skin or hair care treatment to a user comprising:
- determining a scalp type of the user with a method according to any one of claims 1 to 9;
- determining, based on the identified scalp type of the user, the corresponding category of skin and/or hair care compositions and/or skin and/or hair care treatments compatible with the humidity level range and sebum level range of the scalp type of the user; and
- outputting the corresponding category of skin and/or hair care compositions and/or skin and/or hair care treatments.

11. The method according to claim 10, further comprising:
- obtaining a magnified photograph of the region of the scalp of the user;
- upon identifying a number of red portions on the magnified photograph that is above a predetermined threshold, recommend a skin and/or hair care composition comprising a reduced proportion of surfactants adapted for sensitive scalps.

12. The method according to any one of claims 10 or 11, further comprising:
- comparing a current value indicative of the humidity level of the region of the scalp of the user and a current value indicative of the sebum level of a region of the scalp of the user to previously obtained values for the region of the scalp of the user;
- upon determining that the sebum level of the region of the scalp of the user increases, output a recommendation to increase the frequency of hair wash using a hair care product compatible with the scalp type of the user;
- upon determining that the sebum level of the region of the scalp of the user decreases, output a recommendation to use a hair or scalp care product comprising a lower proportion of surfactants.

13. Computer program product comprising instructions for executing a method for determining a scalp type of a user, the method comprising:
- obtaining, as an input, a value indicative of a humidity level of a region of the scalp of the user;
- obtaining, as an input, a value indicative of a sebum level in the region of the scalp of the user;
- comparing a set comprising the obtained value indicative of the humidity level and the value indicative of the sebum level to reference ranges of scalp hydration levels and sebum levels, the reference ranges forming at least five scalp groups, each scalp group being associated with a humidity level range and a sebum level range, each scalp group being further associated with a corresponding category of skin and/or hair care compositions and/or skin and/or hair care treatments compatible with said humidity level range and sebum level range;
wherein the at least five scalp groups comprise:
- a first scalp group (1) corresponding to a hydration level measurable as an upper skin conductivity of 0 µS to 40 µS and a Sebum level measurable as a reduction in translucency of a sebum-absorbing tape film that had been in contact with the scalp of the user between 0% and 20%;
- a second scalp group (2) corresponding to a hydration level measurable as an upper skin conductivity of 0 µS to 80 µS and a Sebum level measurable as a reduction in translucency of a sebum-absorbing tape film that had been in contact with the scalp of the user between 20% and 60%;
- a third scalp group (3) corresponding to a hydration level measurable as an upper skin conductivity of 0 µS to 80 µS and a Sebum level measurable as a reduction in translucency of a sebum-absorbing tape film that had been in contact with the scalp of the user between 60% and 100%;
- a fourth scalp group (4) corresponding to a hydration level measurable as an upper skin conductivity of 40 µS to 80 µS and a Sebum level measurable as a reduction in translucency of a sebum-absorbing tape film that had been in contact with the scalp of the user between 0% and 40%;
- a fifth scalp group (5) corresponding to a hydration level measurable as an upper skin conductivity at or above 80 µS and a Sebum level measurable as a reduction in translucency of a sebum-absorbing tape film that had been in contact with the scalp of the user between 0% and 60%;
- identifying, based on the comparison, a scalp type of the user among the at least five scalp groups; and
- outputting the identified scalp type.

## Patentansprüche

1. Verfahren (10) zur Bestimmung eines Kopfhauttyps eines Benutzers (20), wobei das Verfahren umfasst:
- Ermitteln eines Wertes, der einen Feuchtigkeitsgrad (111) eines Bereichs der Kopfhaut (21) des Benutzers angibt;
- Ermitteln eines Wertes, der einen Talggehalt (112) in dem Bereich der Kopfhaut des Benutzers angibt;
- Vergleichen eines Satzes, der den ermittelten Wert, der den Feuchtigkeitsgrad angibt, und den Wert, der den Talggehalt angibt, mit Referenzbereichen für Kopfhautfeuchtigkeitsgrade und Talggehalte, wobei die Referenzbereiche mindestens fünf Kopfhautgruppen bilden, wobei jede Kopfhautgruppe einem Feuchtigkeitsgradbereich und einem Talggehaltsbereich zugeordnet ist, wobei jede Kopfhautgruppe ferner einer entsprechenden Kategorie von Haut- und/oder Haarpflegezusammensetzungen und/oder Haut- und/oder Haarpflegebehandlungen zugeordnet ist, die mit dem genannten Feuchtigkeitsgradbereich und Talggehaltsbereich kompatibel sind;
wobei die mindestens fünf Kopfhautgruppen umfassen:
- eine erste Kopfhautgruppe (1), die einem Feuchtigkeitsgrad entspricht, der als obere Hautleitfähigkeit von 0 µS bis 40 µS messbar ist, und einem Talggehalt, der als Verringerung der Lichtdurchlässigkeit einer talgabsorbierenden Klebebandfolie, die mit der Kopfhaut des Anwenders in Kontakt stand, zwischen 0 % und 20 % messbar ist;
- eine zweite Kopfhautgruppe (2), die einem Feuchtigkeitsgrad entspricht, der als obere Hautleitfähigkeit von 0 µS bis 80 µS messbar ist, sowie einem Talggehalt, der als Verringerung der Lichtdurchlässigkeit einer talgabsorbierenden Klebebandfolie, die mit der Kopfhaut des Anwenders in Kontakt stand, zwischen 20 % und 60 % messbar ist;
- eine dritte Kopfhautgruppe (3), die einem Feuchtigkeitsgrad entspricht, der als obere Hautleitfähigkeit von 0 µS bis 80 µS messbar ist, sowie einem Talggehalt, der als Verringerung der Lichtdurchlässigkeit einer talgabsorbierenden Klebebandfolie messbar ist, die zwischen 60 % und 100 % mit der Kopfhaut des Anwenders in Kontakt stand;
- eine vierte Kopfhautgruppe (4), die einem Feuchtigkeitsgrad entspricht, der als obere Hautleitfähigkeit von 40 µS bis 80 µS messbar ist, sowie einem Talggehalt, der als Verringerung der Lichtdurchlässigkeit einer talgabsorbierenden Klebebandfolie messbar ist, die zwischen 0 % und 40 % mit der Kopfhaut des Anwenders in Kontakt stand;
- eine fünfte Kopfhautgruppe (5), die einem Feuchtigkeitsgrad entspricht, der als obere Hautleitfähigkeit von 80 µS oder mehr messbar ist, sowie einem Talggehalt v , der als Verringerung der Lichtdurchlässigkeit einer talgabsorbierenden Klebebandfolie messbar ist, die zwischen 0 % und 60 % mit der Kopfhaut des Anwenders in Kontakt stand;
- Identifizieren eines Kopfhauttyps des Benutzers unter den mindestens fünf Kopfhautgruppen (131-135) auf der Grundlage des Vergleichs; und
- Ausgeben des identifizierten Kopfhauttyps.

2. Verfahren nach Anspruch 1, wobei die mindestens fünf Kopfhautgruppen auf der Grundlage von Daten festgelegt werden, die aus den Kopfhäuten von mindestens einhundert Benutzern extrahiert wurden, unter Verwendung mindestens eines der folgenden Verfahren:
- Messungen von biophysikalischen Parameterwerten der Kopfhaut, wobei die biophysikalischen Parameterwerte einen Feuchtigkeitsgradwert und einen Talggradwert für jede Kopfhaut umfassen;
- eine Analyse der Kopfhautmerkmale auf vergrößerten Fotos (430) der Kopfhaut von mindestens hundert Benutzern,

3. Verfahren nach einem der vorstehenden Ansprüche, wobei der Wert, der den Feuchtigkeitsgrad des Bereichs der Kopfhaut des Benutzers angibt, und der Wert, der den Talggehalt des Bereichs der Kopfhaut des Benutzers angibt, unter Verwendung mindestens eines der folgenden Elemente ermittelt werden:
- Messungen von biophysikalischen Parameterwerten des Bereichs der Kopfhaut des Benutzers, wobei die biophysikalischen Parameterwerte einen Feuchtigkeitsgradwert und einen Talggehaltwert des Bereichs der Kopfhaut des Benutzers umfassen;
- einer Analyse von Kopfhautmerkmalen auf vergrößerten Fotografien des Bereichs der Kopfhaut des Benutzers.

4. Verfahren nach einem der Ansprüche 2 bis 3, wobei die Messung der Werte biophysikalischer Parameter der Kopfhaut Messungen der elektrischen Leitfähigkeit oder des elektrischen Widerstands an einer oberen Schicht der Kopfhaut umfasst, um Werte für den Feuchtigkeitsgehalt zu erhalten, sowie Messungen von Änderungen der Lichtdurchlässigkeit von Absorptionsstreifen, die mit der Kopfhaut in Kontakt gebracht werden, um Talggehalte zu erhalten.

5. Verfahren nach einem der Ansprüche 2 bis 4, wobei die Merkmale auf den vergrößerten Fotografien mindestens eines der folgenden umfassen:
- eine Intensität des von dem Bereich der Kopfhaut reflektierten Lichts;
- eine Anzahl von Schuppen (433) auf dem vergrößerten Foto;
- die Anzahl der roten Bereiche (432) auf der vergrößerten Aufnahme, wobei die roten Bereiche auf Entzündungen auf der Kopfhaut hinweisen; und
- eine Menge an fluoreszierendem Licht, das von den Haarfollikeln der Kopfhaut emittiert wird,
wobei jede Kopfhaut unter natürlichem Licht, ultraviolettem Licht und polarisiertem Licht fotografiert wird.

6. Verfahren nach einem der Ansprüche 2 bis 5, das ferner umfasst:
- Festlegung der mindestens fünf Kopfhautgruppen (1-5) auf der Grundlage einer visuellen Beurteilung der Trockenheit und Fettigkeit der Kopfhaut der mindestens einhundert Anwender, wobei die visuelle Beurteilung für jede Kopfhautgruppe der mindestens fünf Kopfhautgruppen einer Beurteilung der Ähnlichkeit der für diese Kopfhautgruppe geeigneten Haut- und/oder Haarpflegezusammensetzungen und/oder Haut- und/oder Haarpflegebehandlungen entspricht.

7. Verfahren nach einem der vorstehenden Ansprüche, wobei die Referenzbereiche auf der Grundlage von Daten festgelegt werden, die von den Kopfhäuten von mindestens einhundert Benutzern gewonnen wurden, wobei das Verfahren ferner für jeden der mindestens einhundert Benutzer umfasst:
- Erfassen vergrößerter Fotografien der Kopfhaut der mindestens einhundert Benutzer;
- Gruppieren der erhaltenen vergrößerten Fotos gemäß den definierten mindestens fünf Kopfhautgruppen;
- für jede der mindestens fünf Kopfhautgruppen die Bestimmung von Kombinationen von Merkmalen, die in den gruppierten vergrößerten Fotos von Kopfhäuten dieser Kopfhautgruppe zu finden sind.

8. Verfahren nach einem der vorstehenden Ansprüche, wobei
- die erste Kopfhautgruppe einer entsprechenden Kategorie von Haut- und/oder Haarpflegemitteln zugeordnet ist, die zwischen 10 % und 18 % Tenside enthalten und einen Zein-Wert von weniger als 0,75 aufweisen;
- die zweite Kopfhautgruppe einer entsprechenden Kategorie von Haut- und/oder Haarpflegemitteln zugeordnet ist, die zwischen 12 % und 20 % Tenside enthalten und einen Zein-Wert von weniger als 1 aufweisen;
- die dritte Kopfhautgruppe ist mit einer entsprechenden Kategorie von Haut- und/oder Haarpflegezusammensetzungen verbunden, die zwischen 14 % und 20 % Tenside enthalten und einen Zein-Wert von weniger als 2,5 aufweisen;
- die vierte Kopfhautgruppe ist mit einer entsprechenden Kategorie von Haut- und/oder Haarpflegezusammensetzungen verbunden, die zwischen 10 % und 18 % Tenside enthalten und einen Zein-Wert von weniger als 2 aufweisen; und
- die fünfte Kopfhautgruppe ist mit einer entsprechenden Kategorie von Haut- und/oder Haarpflegemitteln verbunden, die zwischen 10 % und 18 % Tenside enthalten und einen Zein-Wert von weniger als 1,5 aufweisen.

9. Verfahren nach einem der vorstehenden Ansprüche, wobei:
- die erste Kopfhautgruppe einer entsprechenden Kategorie von Haut- und/oder Haarpflegemitteln zugeordnet ist, die zwischen 0 % und 2 % eines Antischuppenmittels enthalten;
- die zweite Kopfhautgruppe einer entsprechenden Kategorie von Haut- und/oder Haarpflegemitteln zugeordnet ist, die zwischen 0 % und 2 % eines Antischuppenmittels enthalten;
- die dritte Kopfhautgruppe ist einer entsprechenden Kategorie von Haut- und/oder Haarpflegezusammensetzungen zugeordnet, die zwischen 0 % und 1 % eines Antischuppenmittels enthalten;
- die vierte Kopfhautgruppe ist mit einer entsprechenden Kategorie von Haut- und/oder Haarpflegezusammensetzungen verbunden, die kein Mittel gegen Schuppen enthalten; und
- Die fünfte Kopfhautgruppe ist mit einer entsprechenden Kategorie von Haut- und/oder Haarpflegemitteln verbunden, die keine Antischuppenmittel enthalten.

10. Verfahren zum Empfehlen eines Haut- oder Haarpflegeprodukts und/oder einer Haut- oder Haarpflegebehandlung an einen Benutzer, umfassend:
- Bestimmung eines Kopfhauttyps des Benutzers mit einem Verfahren gemäß einem der Ansprüche 1 bis 9;
- Ermitteln der entsprechenden Kategorie von Haut- und/oder Haarpflegezusammensetzungen und/oder Haut- und/oder Haarpflegebehandlungen, die mit dem Feuchtigkeits- und Talggehaltsbereich des Kopfhauttyps des Benutzers kompatibel sind, auf der Grundlage des ermittelten Kopfhauttyps des Benutzers; und
- Ausgeben der entsprechenden Kategorie von Haut- und/oder Haarpflegezusammensetzungen und/oder Haut- und/oder Haarpflegebehandlungen.

11. Verfahren nach Anspruch 10, das ferner umfasst:
- Erhalten eines vergrößerten Fotos des Bereichs der Kopfhaut des Benutzers;
- bei Feststellung einer Anzahl roter Bereiche auf der vergrößerten Aufnahme, die über einem vorbestimmten Schwellenwert liegt, Empfehlung einer Haut- und/oder Haarpflegezusammensetzung, die einen reduzierten Anteil an Tensiden enthält und für empfindliche Kopfhaut geeignet ist.

12. Verfahren nach einem der Ansprüche 10 oder 11, das ferner umfasst:
- Vergleichen eines aktuellen Wertes, der den Feuchtigkeitsgrad des Bereichs der Kopfhaut des Benutzers angibt, und eines aktuellen Wertes, der den Talggehalt eines Bereichs der Kopfhaut des Benutzers angibt, mit zuvor ermittelten Werten für den Bereich der Kopfhaut des Benutzers;
- bei Feststellung, dass der Talggehalt des Bereichs der Kopfhaut des Benutzers ansteigt, Ausgabe einer Empfehlung, die Häufigkeit der Haarwäsche unter Verwendung eines mit dem Kopfhauttyp des Benutzers kompatiblen Haarpflegeprodukts zu erhöhen;
- bei Feststellung, dass der Talggehalt des Bereichs der Kopfhaut des Benutzers abnimmt, Ausgabe einer Empfehlung zur Verwendung eines Haar- oder Kopfhautpflegeprodukts, das einen geringeren Anteil an Tensiden enthält.

13. Computerprogrammprodukt, das Anweisungen zur Ausführung eines Verfahrens zur Bestimmung eines Kopfhauttyps eines Benutzers umfasst, wobei das Verfahren Folgendes umfasst:
- Erfassen eines Wertes, der den Feuchtigkeitsgrad eines Bereichs der Kopfhaut des Benutzers angibt, als Eingabe;
- Erfassen eines Wertes als Eingabe, der einen Talggehalt in dem Bereich der Kopfhaut des Benutzers angibt;
- Vergleichen eines Satzes, der den ermittelten Wert, der den Feuchtigkeitsgrad angibt, und den Wert, der den Talggehalt angibt, mit Referenzbereichen für den Feuchtigkeitsgrad und den Talggehalt der Kopfhaut, wobei die Referenzbereiche mindestens fünf Kopfhautgruppen bilden, wobei jede Kopfhautgruppe einem Feuchtigkeitsgradbereich und einem Talggehaltsbereich zugeordnet ist, wobei jede Kopfhautgruppe ferner einer entsprechenden Kategorie von Haut- und/oder Haarpflegezusammensetzungen und/oder Haut- und/oder Haarpflegebehandlungen zugeordnet ist, die mit dem genannten Feuchtigkeitsgradbereich und Talggehaltsbereich kompatibel sind;
wobei die mindestens fünf Kopfhautgruppen umfassen:
- eine erste Kopfhautgruppe (1), die einem Feuchtigkeitsgrad entspricht, der als obere Hautleitfähigkeit von 0 µS bis 40 µS messbar ist, und einem Talggehalt, der als Verringerung der Lichtdurchlässigkeit einer talgabsorbierenden Klebebandfolie, die mit der Kopfhaut des Anwenders in Kontakt stand, zwischen 0 % und 20 % messbar ist;
- eine zweite Kopfhautgruppe (2), die einem Feuchtigkeitsgrad entspricht, der als obere Hautleitfähigkeit von 0 µS bis 80 µS messbar ist, sowie einem Talggehalt, der als Verringerung der Lichtdurchlässigkeit einer talgabsorbierenden Klebebandfolie, die mit der Kopfhaut des Anwenders in Kontakt stand, zwischen 20 % und 60 % messbar ist;
- eine dritte Kopfhautgruppe (3), die einem Feuchtigkeitsgrad entspricht, der als obere Hautleitfähigkeit von 0 µS bis 80 µS messbar ist, sowie einem Talggehalt, der als Verringerung der Lichtdurchlässigkeit einer talgabsorbierenden Klebebandfolie messbar ist, die zwischen 60 % und 100 % mit der Kopfhaut des Anwenders in Kontakt stand;
- eine vierte Kopfhautgruppe (4), die einem Feuchtigkeitsgrad entspricht, der als obere Hautleitfähigkeit von 40 µS bis 80 µS messbar ist, sowie einem Talggehalt, der als Verringerung der Lichtdurchlässigkeit einer talgabsorbierenden Klebebandfolie messbar ist, die zwischen 0 % und 40 % mit der Kopfhaut des Anwenders in Kontakt stand;
- eine fünfte Kopfhautgruppe (5), die einem Feuchtigkeitsgrad entspricht, der als obere Hautleitfähigkeit von 80 µS oder mehr messbar ist, sowie einem Talggehalt, der als Verringerung der Lichtdurchlässigkeit einer talgabsorbierenden Klebebandfolie, die mit der Kopfhaut des Benutzers in Kontakt stand, zwischen 0 % und 60 % messbar ist;
- Identifizieren eines Kopfhauttyps des Benutzers unter den mindestens fünf Kopfhautgruppen auf der Grundlage des Vergleichs; und
- Ausgeben des identifizierten Kopfhauttyps.

## Revendications

1. Procédé (10) permettant de déterminer le type de cuir chevelu d'un utilisateur (20), le procédé comprenant :
- l'obtention d'une valeur indicative d'un niveau d'humidité (111) d'une région du cuir chevelu (21) de l'utilisateur ;
- l'obtention d'une valeur indicative d'un niveau de sébum (112) dans la zone du cuir chevelu de l'utilisateur ;
- comparer un ensemble comprenant la valeur obtenue indicative du niveau d'humidité et la valeur indicative du niveau de sébum à des plages de référence de niveaux d'hydratation du cuir chevelu et de niveaux de sébum, les plages de référence formant au moins cinq groupes de cuir chevelu, chaque groupe de cuir chevelu étant associé à une plage de niveaux d'humidité et à une plage de niveaux de sébum, chaque groupe de cuir chevelu étant en outre associé à une catégorie correspondante de compositions de soins de la peau et/ou des cheveux et/ou de traitements de soins de la peau et/ou des cheveux compatibles avec ladite plage de niveaux d'humidité et ladite plage de niveaux de sébum ;
dans lequel les au moins cinq groupes de cuir chevelu comprennent :
- un premier groupe de cuir chevelu (1) correspondant à un niveau d'hydratation mesurable par une conductivité cutanée supérieure comprise entre 0 µS et 40 µS et à un niveau de sébum mesurable par une réduction de la translucidité d'un film de ruban absorbant le sébum ayant été en contact avec le cuir chevelu de l'utilisateur comprise entre 0 % et 20 % ;
- un deuxième groupe de cuir chevelu (2) correspondant à un niveau d'hydratation mesurable par une conductivité cutanée supérieure comprise entre 0 µS et 80 µS et à un niveau de sébum mesurable par une réduction de la translucidité d'un film adhésif absorbant le sébum qui a été en contact avec le cuir chevelu de l'utilisateur comprise entre 20 % et 60 % ;
- un troisième groupe de cuir chevelu (3) correspondant à un niveau d'hydratation mesurable par une conductivité cutanée supérieure comprise entre 0 µS et 80 µS et à un niveau de sébum mesurable par une réduction de la translucidité d'un film adhésif absorbant le sébum qui avait été en contact avec le cuir chevelu de l'utilisateur entre 60 % et 100 % ;
- un quatrième groupe de cuir chevelu (4) correspondant à un niveau d'hydratation mesurable par une conductivité cutanée supérieure comprise entre 40 µS et 80 µS et à un niveau de sébum mesurable par une réduction de la translucidité d'un film adhésif absorbant le sébum qui a été en contact avec le cuir chevelu de l'utilisateur entre 0 % et 40 % ;
- un cinquième groupe de cuir chevelu (5) correspondant à un niveau d'hydratation mesurable par une conductivité cutanée supérieure ou égale à 80 µS et à un niveau de sébum mesurable par une réduction de la translucidité d'un film adhésif absorbant le sébum ayant été en contact avec le cuir chevelu de l'utilisateur entre 0 % et 60 % ;
- identifier, sur la base de la comparaison, un type de cuir chevelu de l'utilisateur parmi les au moins cinq groupes de cuir chevelu (131-135) ; et
- afficher le type de cuir chevelu identifié.

2. Procédé selon la revendication 1, dans lequel les au moins cinq groupes de cuir chevelu sont établis sur la base de données extraites du cuir chevelu d'au moins cent utilisateurs à l'aide d'au moins l'un des éléments suivants :
- des mesures des valeurs des paramètres biophysiques des cuirs chevelus, les valeurs des paramètres biophysiques comprenant une valeur de niveau d'hydratation et une valeur de niveau de sébum pour chaque cuir chevelu ;
- une analyse des caractéristiques du cuir chevelu à partir de photographies agrandies (430) du cuir chevelu d'au moins cent utilisateurs,

3. Procédé selon l'une quelconque des revendications précédentes, dans lequel la valeur indicative du niveau d'humidité de la région du cuir chevelu de l'utilisateur et la valeur indicative du niveau de sébum de la région du cuir chevelu de l'utilisateur sont obtenues en utilisant au moins l'un des éléments suivants :
- des mesures des valeurs des paramètres biophysiques de la région du cuir chevelu de l'utilisateur, les valeurs des paramètres biophysiques comprenant une valeur du niveau d'hydratation et une valeur du niveau de sébum de la région du cuir chevelu de l'utilisateur ;
- une analyse des caractéristiques du cuir chevelu sur des photographies agrandies de la région du cuir chevelu de l'utilisateur.

4. Procédé selon l'une quelconque des revendications 2 à 3, dans lequel la mesure des valeurs des paramètres biophysiques du cuir chevelu comprend des mesures de conductivité électrique ou des mesures de résistance électrique sur une couche supérieure du cuir chevelu afin d'obtenir des valeurs de niveaux d'hydratation, et des mesures des variations de translucidité de bandelettes absorbantes mises en contact avec le cuir chevelu afin d'obtenir des niveaux de sébum.

5. Procédé selon l'une quelconque des revendications 2 à 4, dans lequel les caractéristiques sur les photographies agrandies comprennent au moins l'un des éléments suivants :
- une intensité de lumière réfléchie par la région du cuir chevelu ;
- un nombre de pellicules (433) sur la photographie agrandie ;
- un nombre de zones rouges (432) sur la photographie agrandie, les zones rouges étant indicatives d'inflammations sur le cuir chevelu ; et
- une quantité de lumière fluorescente émise par les follicules du cuir chevelu, chaque cuir chevelu étant photographié sous des conditions de lumière naturelle, de lumière ultraviolette et de lumière polarisée.

6. Procédé selon l'une quelconque des revendications 2 à 5, comprenant en outre :
- la définition d'au moins cinq groupes de cuir chevelu (1-5) sur la base d'une évaluation visuelle de la sécheresse et de l'aspect gras des cuirs chevelus d'au moins cent utilisateurs, l'évaluation visuelle correspondant, pour chaque groupe de cuir chevelu parmi les au moins cinq groupes de cuir chevelu, à une évaluation de la similitude des compositions de soins de la peau et/ou des cheveux et/ou des traitements de soins de la peau et/ou des cheveux adaptés audit groupe de cuir chevelu.

7. Procédé selon l'une quelconque des revendications précédentes, dans lequel les plages de référence sont établies sur la base de données extraites du cuir chevelu d'au moins cent utilisateurs, le procédé comprenant en outre, pour chacun desdits au moins cent utilisateurs :
- l'obtention de photographies agrandies du cuir chevelu desdits au moins cent utilisateurs ;
- le regroupement des photographies agrandies obtenues selon les au moins cinq groupes de cuir chevelu définis ;
- pour chaque groupe de cuir chevelu parmi les au moins cinq groupes de cuir chevelu, déterminer des combinaisons de caractéristiques présentes dans les photographies agrandies regroupées de cuirs chevelus appartenant audit groupe de cuir chevelu.

8. Procédé selon l'une quelconque des revendications précédentes, dans lequel
- le premier groupe de cuirs chevelus est associé à une catégorie correspondante de compositions de soins de la peau et/ou des cheveux comprenant entre 10 % et 18 % de tensioactifs, et ayant une valeur Zein inférieure à 0,75 ;
- le deuxième groupe de cuir chevelu est associé à une catégorie correspondante de compositions de soins de la peau et/ou des cheveux comprenant entre 12 % et 20 % de tensioactifs, et ayant une valeur de Zein inférieure à 1 ;
- le troisième groupe de cuir chevelu est associé à une catégorie correspondante de compositions de soins de la peau et/ou des cheveux comprenant entre 14 % et 20 % de tensioactifs, et présentant une valeur de Zein inférieure à 2,5 ;
- le quatrième groupe de cuir chevelu est associé à une catégorie correspondante de compositions de soins de la peau et/ou des cheveux comprenant entre 10 % et 18 % de tensioactifs, et présentant une valeur Zein inférieure à 2 ; et
- le cinquième groupe de cuir chevelu est associé à une catégorie correspondante de compositions de soins de la peau et/ou des cheveux comprenant entre 10 % et 18 % de tensioactifs, et présentant une valeur Zein inférieure à 1,5.

9. Procédé selon l'une quelconque des revendications précédentes, dans lequel :
- le premier groupe de cuir chevelu est associé à une catégorie correspondante de compositions de soins de la peau et/ou des cheveux comprenant entre 0 % et 2 % d'un agent antipelliculaire ;
- le deuxième groupe de cuir chevelu est associé à une catégorie correspondante de compositions de soins de la peau et/ou des cheveux comprenant entre 0 % et 2 % d'un agent antipelliculaire ;
- le troisième groupe de cuir chevelu est associé à une catégorie correspondante de compositions de soins de la peau et/ou des cheveux comprenant entre 0 % et 1 % d'un agent antipelliculaire ;
- le quatrième groupe de cuir chevelu est associé à une catégorie correspondante de compositions de soins de la peau et/ou des cheveux ne contenant pas d'agent antipelliculaire ; et
- le cinquième groupe de cuir chevelu est associé à une catégorie correspondante de compositions de soins de la peau et/ou des cheveux ne contenant pas d'agent antipelliculaire.

10. Procédé pour recommander à un utilisateur un produit de soin de la peau ou des cheveux et/ou un traitement de soin de la peau ou des cheveux, comprenant :
- la détermination du type de cuir chevelu de l'utilisateur à l'aide d'un procédé selon l'une quelconque des revendications 1 à 9 ;
- déterminer, sur la base du type de cuir chevelu identifié de l'utilisateur, la catégorie correspondante de compositions de soins de la peau et/ou des cheveux et/ou de traitements de soins de la peau et/ou des cheveux compatibles avec la plage de taux d'humidité et la plage de taux de sébum du type de cuir chevelu de l'utilisateur ; et
- fournir la catégorie correspondante de compositions de soins de la peau et/ou des cheveux et/ou de traitements de soins de la peau et/ou des cheveux.

11. Procédé selon la revendication 10, comprenant en outre :
- l'obtention d'une photographie agrandie de la région du cuir chevelu de l'utilisateur ;
- lorsqu'un nombre de zones rouges sur la photographie agrandie est supérieur à un seuil prédéterminé, recommander une composition de soin de la peau et/ou des cheveux comprenant une proportion réduite de tensioactifs adaptée aux cuirs chevelus sensibles.

12. Procédé selon l'une quelconque des revendications 10 ou 11, comprenant en outre :
- la comparaison d'une valeur actuelle indicative du niveau d'humidité de la région du cuir chevelu de l'utilisateur et d'une valeur actuelle indicative du niveau de sébum d'une région du cuir chevelu de l'utilisateur avec des valeurs obtenues précédemment pour la région du cuir chevelu de l'utilisateur ;
- lorsqu'il est déterminé que le niveau de sébum de la région du cuir chevelu de l'utilisateur augmente, émettre une recommandation visant à augmenter la fréquence des lavages de cheveux à l'aide d'un produit de soin capillaire compatible avec le type de cuir chevelu de l'utilisateur ;
- lorsqu'il est déterminé que le niveau de sébum de la zone du cuir chevelu de l'utilisateur diminue, émettre une recommandation d'utiliser un produit de soin des cheveux ou du cuir chevelu comprenant une proportion plus faible de tensioactifs.

13. Produit logiciel comprenant des instructions pour exécuter un procédé de détermination du type de cuir chevelu d'un utilisateur, le procédé comprenant :
- l'obtention, en entrée, d'une valeur indicative du niveau d'humidité d'une zone du cuir chevelu de l'utilisateur ;
- l'obtention, en entrée, d'une valeur indicative d'un niveau de sébum dans la région du cuir chevelu de l'utilisateur ;
- comparer un ensemble comprenant la valeur obtenue indicative du niveau d'humidité et la valeur indicative du niveau de sébum à des plages de référence de niveaux d'hydratation du cuir chevelu et de niveaux de sébum, les plages de référence formant au moins cinq groupes de cuir chevelu, chaque groupe de cuir chevelu étant associé à une plage de niveaux d'humidité et à une plage de niveaux de sébum, chaque groupe de cuir chevelu étant en outre associé à une catégorie correspondante de compositions de soins de la peau et/ou des cheveux et/ou de traitements de soins de la peau et/ou des cheveux compatibles avec ladite plage de niveaux d'humidité et ladite plage de niveaux de sébum ;
dans lequel les au moins cinq groupes de cuir chevelu comprennent :
- un premier groupe de cuir chevelu (1) correspondant à un niveau d'hydratation mesurable par une conductivité cutanée supérieure comprise entre 0 µS et 40 µS et à un niveau de sébum mesurable par une réduction de la translucidité d'un film de ruban absorbant le sébum ayant été en contact avec le cuir chevelu de l'utilisateur comprise entre 0 % et 20 % ;
- un deuxième groupe de cuir chevelu (2) correspondant à un niveau d'hydratation mesurable par une conductivité cutanée supérieure comprise entre 0 µS et 80 µS et à un niveau de sébum mesurable par une réduction de la translucidité d'un film adhésif absorbant le sébum qui a été en contact avec le cuir chevelu de l'utilisateur comprise entre 20 % et 60 % ;
- un troisième groupe de cuir chevelu (3) correspondant à un niveau d'hydratation mesurable par une conductivité cutanée supérieure comprise entre 0 µS et 80 µS et à un niveau de sébum mesurable par une réduction de la translucidité d'un film adhésif absorbant le sébum qui avait été en contact avec le cuir chevelu de l'utilisateur entre 60 % et 100 % ;
- un quatrième groupe de cuir chevelu (4) correspondant à un niveau d'hydratation mesurable par une conductivité cutanée supérieure comprise entre 40 µS et 80 µS et à un niveau de sébum mesurable par une réduction de la translucidité d'un film adhésif absorbant le sébum qui a été en contact avec le cuir chevelu de l'utilisateur entre 0 % et 40 % ;
- un cinquième groupe de cuir chevelu (5) correspondant à un niveau d'hydratation mesurable par une conductivité cutanée superficielle égale ou supérieure à 80 µS et à un niveau de sébum mesurable par une réduction de la translucidité d'un film adhésif absorbant le sébum qui a été en contact avec le cuir chevelu de l'utilisateur comprise entre 0 % et 60 % ;
- identifier, sur la base de la comparaison, un type de cuir chevelu de l'utilisateur parmi les au moins cinq groupes de cuir chevelu ; et
- afficher le type de cuir chevelu identifié.
